# FASCICULE DE BREVET EUROPEEN

(11) **EP 0 649 906 B1**
(45) Date de publication et mention de la délivrance du brevet: **07.01.2004**
(21) Numéro de dépôt: 94110776.5
(22) Date de dépôt: 06.10.1989
(51) Int. Cl.: C12N 15/55, C12N 9/80, C12Q 1/68, G01N 33/569, A61K 39/106

(54) **Séquence de nucléotides codant pour une protéine à activité uréasique**
Nukleotidsequenzen, die für ein Protein mit Ureaseaktivität kodieren
Nucleotide sequences coding for a protein with urease activity

(30) Priorité: 06.10.1988 FR 8813135
(43) Date de publication de la demande: 26.04.1995
(62) Demande divisionnaire de: 89402764.8
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: Labigne, Agnès, F-91440 Bures Sur Yvette (FR)
(74) Mandataire: Gutmann, Ernest

(56) Documents cités:
- EP-A- 0 204 438
- WO-A-86/04422
- WO-A-87/01119
- CHEMICAL ABSTRACTS, vol. 109, no. 7, 15 Août 1988, Columbus, Ohio, US; abstract no. 50580d, H. MOBLEY ET AL. 'Characterization of urease from Campylobacter pylori.' page 289 ; & J. CLIN.MICROBIOL., vol.26, no.5, 1988 pages 831 - 836
- CHEMICAL ABSTRACTS, vol. 110, no. 1, 2 Janvier 1989, Columbus, Ohio, US; abstract no. 3472f, R.L.FERRERO ET AL 'The urease enzymes of Campylobacter pylori and a related bacterium.' page 323 ; & J.MED.MICROBIOL., vol.27, no.1, 1988 pages 33 - 40

## Description

L'invention a pour objet des séquences de nucléotides codant pour une uréase telle qu'exprimée naturellement chez Campylobacter pylori et ses applications biologiques, notamment pour la détection de C.pylori chez l'homme ou dans l'environnement.

C.pylori est une bactérie à gram négatif retrouvée à ce jour exclusivement à la surface de la muqueuse de la partie antrale de l'estomac chez l'homme, et plus particulièrement autour des lésions et des cratères des ulcères gastriques et duodénaux. 25% de la population est porteuse de C.pylori: 8% présentent une maladie ulcéreuse, 9% souffrent de dyspepsie non-ulcéreuse et 8% sont porteurs asymptomatiques.

Tous les C.pylori isolés et décrits à ce jour, possèdent les trois propriétés suivantes:

Les bactéries produisent une uréase dotée d'une grande activité.

Elles adhèrent très fortement aux cellules épithéliales de la muqueuse gastrique, cette propriété se traduisant in vitro par une très forte adhésion aux cellules Hela.

Les C.pylori produisent et libèrent une enzyme présentant une activité protéolytique conduisant à la dégradation du mucus et donc à un affaiblissement de la barrière naturelle protégeant la muqueuse gastrique.

La détection de C.pylori in situ, chez l'homme et dans l'environnement et l'étude de son pouvoir pathogène - elle est considérée comme responsable des gastrites actives chez l'homme - se heurtent toutefois aux difficultés de culture de cet organisme.

Sa croissance est très lente (6 à 7 jours sur milieu gélosé au sang), et doit s'effectuer dans des conditions de microaérophilie, l'oxygène de l'air étant toxique.

La recherche de moyens de détection de cette bactérie a conduit les inventeurs à identifier les gènes responsables chez C.pylori de la production d'uréase.

La détermination de la séquence de nucléotides d'un fragment intragénique a permis de disposer d'un outil utilisable comme sonde de détection pour l'identification spécifique des C.pylori.

L'invention a donc pour but de fournir de nouvelles séquences de nucléotides capables de coder pour des protéines à activité uréasique telles qu'exprimées chez C.pylori.

Elle a également pour but de fournir des fragments de cette séquence utilisables comme sondes de détection de C.pylori.

L'invention vise, en outre, à fournir une protéine à activité uréasique telle qu'exprimée chez C.pylori, de pureté élevée, et des fragments de cette protéine.

La séquence de nucléotides selon l'invention est caractérisée en ce qu'elle comprend au moins une partie d'une séquence codant pour une protéine à activité uréase telle qu'exprimée chez C.pylori.

L'invention a plus particulièrement pour objet une séquence de nucléotides capable de s'hybrider avec des gènes codant pour une protéine à activité uréase telle qu'exprimée chez C.pylori, dans les conditions suivantes: 68°C, 6 x SSC (1 x SSC est constitué de 0,15M de NaCl et 0,015M de citrate de sodium, pH7), en milieu Denhardt (1% Ficoll, 1% polyvinylpyrrolidone, 1% de sérum albumine de boeuf).

Selon un autre aspect de l'invention, la séquence de nucléotides est caractérisée en ce qu'elle porte l'information requise pour la production d'une protéine à activité uréase ou de ses fragments, capables de former un complexe immunologique avec des anticorps dirigés respectivement contre une protéine d'activité uréase telle qu'exprimée chez C.pylori ou contre des fragments de cette dernière.

La séquence selon l'invention est également caractérisée en ce qu'elle comprend au moins une partie d'un fragment d'environ 8kb correspondant au fragment de restriction EcoRI (ClaI, BamHI)-PstI (HindIII).

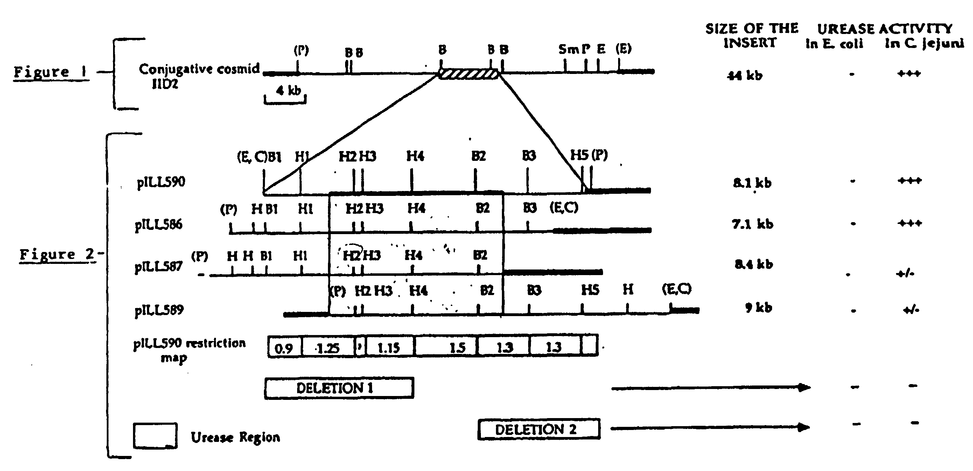

La carte de restriction enzymatique de ce fragment est représentée sur la figure 2.

Une séquence plus spécialement préférée comprend au moins une partie d'un fragment d'environ 4,2kb (±5%) délimité par les nucléotides d'extrémité situés respectivement à une distance d'environ 0,55kb en amont du site H2 représenté sur la figure 2, et d'environ 0,7kb en aval du site B1 représenté sur la figure 2.

Les séquences constituées par ce fragment portent l'information nécessaire pour l'expression d'une protéine à activité uréase telle qu'exprimée naturellement chez C.pylori.

L'expression de ces séquences peut intervenir chez tout autre microorganisme capable d'exploiter à son profit l'information portée par ces séquences, notamment chez une souche de Campylobacter naturellement déficiente en gènes codant pour la production d'une protéine à activité uréase (ou encore souche de Campylobacter uréase-).

Selon encore un autre aspect, l'invention vise une séquence recombinante comprenant l'une des séquences définies ci-dessus, le cas échéant associée à un promoteur capable de contrôler la transcription de la séquence et une séquence d'ADN codant pour des signaux de terminaison de la transcription.

La présente demande décrit également des vecteurs recombinants d'expression et de clonage, capables de transformer une cellule hôte appropriée, comportant au moins une partie d'une séquence de nucléotides telle que définie ci-dessus sous le contrôle d'éléments de régulation permettant son expression.

Des vecteurs recombinants préférés renferment au moins une partie de la séquence d'environ 4,2kb évoquée plus haut.

Les souches de micro-organismes, transformées sont également décrites dans le cadre de la présente demande. Ces souches comportent l'une des séquences de nucléotides définies ci-dessus ou encore un vecteur recombinant tel que défini précédemment.

La souche E.coli S17-1 déposée le 16 Août 1988 sous le n° I-795 à la Collection Nationale de Culture de Microorganismes (C.N.C.M.) de l'Institut Pasteur à PARIS (France) porte le plasmide pILL590 de 16,3kb environ qui comprend le fragment de restriction d'environ 8kb EcoRI (ClaI, BamHI)-PstI (Hind III) évoqué plus haut.

L'invention vise en outre une protéine présentant une activité uréase du type de celle exprimée naturellement chez C.pylori ainsi que les fragments peptidiques de cette protéine.

La protéine de l'invention, et ses fragments, correspondent selon le code génétique universel, aux séquences de nucléotides définies ci-dessus, en particulier à au moins une partie de la séquence d'environ 4,2kb.

La protéine de l'invention est également caractérisée en ce qu'il s'agit d'une protéine telle qu'obtenue par transformation de cellules hôtes au moyen d'un vecteur recombinant comme défini ci-dessus, mise en culture, dans un milieu approprié, des cellules hôtes transformées et récupération de la protéine à partir de ces cellules ou directement à partir du milieu de culture. La production d'uréase ou de fragments de cette dernière par ce procédé, fait également partie de l'invention.

La protéine de l'invention et ses fragments, qui peuvent être également obtenus par synthèse chimique, présentent avantageusement un degré de pureté élevé et sont utilisés pour former, selon les techniques classiques, des anticorps polyclonaux.

De tels anticorps polyclonaux, ainsi que les anticorps monoclonaux capables de reconnaître spécifiquement la protéine ci-dessus et ses fragments sont également visés par l'invention.

Les séquences de nucléotides définies ci-dessus sont obtenues selon les techniques classiques du génie génétique par clonage et identification des gènes responsables de la synthèse d'une protéine à activité uréase chez C.pylori.

L'invention vise également les applications biologiques des séquences de nucléotides, des protéines correspondantes et des anticorps monoclonaux ou polyclonaux.

Ces applications comprennent l'élaboration, à partir de fragments intragéniques, de sondes pour la détection de C.pylori. Cette élaboration comprend, notamment, la dénaturation des séquences double-brin pour obtenir une séquence monobrin utilisable en tant que sonde.

Les essais réalisés avec de tels fragments pour détecter la présence éventuelle de séquences complémentaires chez divers Campylobacter et chez des microorganismes appartenant à des genres différents ont mis en évidence la grande spécificité de ces fragments.

L'invention vise donc des sondes de détection caractérisées en ce qu'elles comprennent au moins une partie d'une séquence de nucléotides définie ci-dessus.

Toute sonde ne se distinguant de la précédente, au niveau de sa séquence de nucléotides, que par des substitutions ou altérations de nucléotides n'entraînant pas de modification de ses propriétés d'hybridation avec le génome de C.pylori, entre dans le cadre de l'invention.

Le fragment d'ADN utilisé comme sonde comporte un nombre de nucléotides suffisant pour obtenir la spécificité requise et la formation d'un hybride stable.

Il est possible d'utiliser des fragments atteignant plusieurs kb, des résultats de haute spécificité étant cependant également obtenus avec des fragments plus courts d'environ 25 à 40 nucléotides.

Des sondes appropriées pour ce type de détection sont avantageusement marquées par un élément radio-actif ou tout autre groupe permettant sa reconnaissance à l'état hybridé avec la préparation renfermant l'ADN à étudier.

Selon les techniques classiques, ces sondes sont mises en contact avec un échantillon biologique renfermant des bactéries, ou directement avec ces bactéries ou leurs acides nucléiques, dans des conditions autorisant l'hybridation éventuelle de la séquence de nucléotides de la sonde avec une séquence complémentaire, éventuellement contenue dans le produit testé.

On peut, par exemple, mettre en oeuvre la méthode d'hybridation sur taches. Cette méthode comporte après dénaturation de l'ADN préalablement obtenu à partir de bactéries provenant de biopsies antrales, le dépôt d'une quantité aliquote de cet ADN sur des membranes de nitrocellulose, l'hybridation de chaque membrane dans les conditions usuelles avec la sonde et la détection, de manière classique, de l'hybride formé.

On peut aussi utiliser une méthode d'hybridation sur réplique, selon la technique de Southern. Cette méthode comprend la séparation électrophorétique en gel d'agarose des fragments d'ADNs engendrés après traitement de l'ADN par des enzymes de restriction, le transfert après dénaturation alcaline sur des membranes appropriées et leur hybridation avec la sonde dans les conditions usuelles. Il n'est pas toujours nécessaire de procéder à l'expression préalable de l'ADN. Il suffit que l'ADN soit rendu accessible à la sonde.

La détection pour l'identification spécifique des C.pylori peut être également réalisée par des techniques d'amplification de l'ADN (PCR). Ces techniques sont décrites en particulier dans les brevets US.4683202 et 4683195 au nom de Cetus Corporation.

Pour la mise en oeuvre de ces techniques, on utilise deux amorces de 10 à 40 nucléotides, avantageusement d'environ une vingtaine de nucléotides, ces amorces étant comprises dans l'une des séquences de nucléotides définies ci-dessus ou susceptibles de s'hybrider avec une partie d'une des séquences nucléotidiques définies ci-dessus, ou de leurs séquences complémentaires. De manière avantageuse, ces amorces sont distantes d'environ 200 à 250 nucléotides lorsqu'elles sont hybridées (ou liées), aux brins de l'ADN à amplifier. l'une des séquences est capable de se lier à une séquence de nucléotides d'un des brins de fragments d'ADN à amplifier, cette séquence étant située au niveau d'une extrémité de ce fragment (par exemple l'extrémité 5') ; l'autre séquence est capable de se lier à une séquence de nucléotides du deuxième brin du fragment d'ADN à amplifier, cette dernière séquence étant située au niveau de l'extrémité de ce fragment opposée à celle sus-mentionnée (cette dernière extrémité étaént encore désignée par extrémité 3' dans l'exemple proposé).

Des amorces préférées sont comprises dans la séquence de nucléotides du fragment de restriction H2-H3 de la figure 2, et sont distantes d'environ 200 à 250 nucléotides.

Il s'agit, en particulier, des fragments respectivement à chaque extrémité de cette séquence de restriction HindIII-HindIII.

Un procédé de dépistage in vitro de la présence éventuelle de C.pylori dans un échantillon biologique comprend les étapes suivantes:
- éventuellement l'amplification préalable de la quantité de séquences de nucléotides susceptibles d'être contenues dans l'échantillon, par mise en contact de cet échantillon avec des amorces telles que décrites ci-dessus, ces amorces étant susceptibles respectivement de se lier d'une part à l'extrémité 5' d'un brin de ladite séquence de nucléotides et d'autre part, à l'extrémité 3' de l'autre brin de ladite séquence de nucléotides, cette mise en contact étant suivie d'une étape d'amplification génique en présence d'une ADN polymérase et des quatre nucléosides triphosphates dATP, dCTP, dGTP, dTTP, ces opérations d'hybridation des amorces et d'amplification génique étant répétées plusieurs fois,
- la mise en contact de l'échantillon biologique en question avec une sonde nucléotidique selon l'invention, dans des conditions permettant la production d'un complexe d'hybridation formé entre la sonde et la séquence de nucléotides,
- la détection du complexe d'hybridation.

L'invention fournit ainsi des outils permettant de détecter rapidement C.pylori, in situ, et dans l'environnement avec une grande spécificité, sans avoir à pratiquer de cultures.

De tels outils de détection sont particulièrement utiles pour étudier le réservoir naturel de ces bactéries, les méthodes de transmission, de circulation et de contamination. De plus, dans le diagnostic in vitro pratiqué sur les biopsies, l'utilisation de ces sondes permet un gain de temps considérable par rapport aux techniques actuelles qui nécessitent de plus une technologie ne pouvant être réalisée que dans des services spécialisés, à savoir, des techniques bactériologiques ou utilisant la microscopie électronique.

Des résultats reproductibles ont été obtenus en utilisant comme sonde intragénique le fragment d'environ 8kb défini ci-dessus.

Compte tenu de leur spécificité à l'égard de C.pylori, ces sondes constituent également un outil de grand intérêt.

Pour étudier le pouvoir pathogène de C.pylori de façon à prévenir l'infection, il est ainsi possible d'étudier le rôle joué par C.pylori dans le développement des maladies ulcéreuses et d'identifier les déterminants génétiques qui semblent impliqués dans le pouvoir pathogène de cet organisme en vue de créer in vitro des mutants isogéniques de C.pylori, c.a.d. des bactéries modifiées génétiquement dans chacun des déterminants qui semblent jouer un rôle dans la pathogénèse de l'infection, mutants qui pourront être testés dans des modèles animaux.

L'invention concerne également la détection du polymorphisme des gènes codant pour l'uréase chez C.pylori à l'aide des sondes nucléotidiques définies ci-dessus et d'enzymes de restriction appropriées. Les conditions dans lesquelles cette détection est réalisée sont plus particulièrement décrites dans l'article de Gusella J.F. dans J. of Clin. - investigations (1986), 77, 1723-1726.

L'invention vise également les applications immunologiques de la protéine codée, plus spécialement pour l'élaboration d'antisérum spécifique ainsi que d'anticorps polyclonaux et monoclonaux. Les anticorps polyclonaux sont formés selon les techniques classiques par injection de la protéine à des animaux, récupération des antisérums, puis des anticorps à partir des antisérums par exemple par chromatographie d'affinité.

Les anticorps monoclonaux sont produits de manière habituelle en fusionnant des cellules myélomes avec des cellules de rate d'animaux préalablement immunisés à l'aide des protéines de l'invention.

L'invention vise, en outre, un procédé de dépistage in vitro de la présence éventuelle de C.pylori dans un échantillon biologique susceptible de le contenir. Ce procédé est caractérisé en ce qu'il comprend:
- la mise en contact de l'échantillon avec un anticorps selon l'invention, dans des conditions permettant la production d'un complexe immunologique formé entre tout ou partie de la protéine à activité uréase produite par C.pylori et cet anticorps, et
- la détection du complexe immunologique.

Pour la mise en oeuvre des méthodes de dépistage in vitro, considérées ci-dessus basées sur l'utilisation de sondes nucléotidiques, on a recours avantageusement à des nécessaires ou kits comprenant:
- le cas échéant, au moins un couple d'amorces selon l'invention,
- une quantité déterminée d'une sonde nucléotidique selon l'invention,
- avantageusement, un milieu approprié respectivement à la formation d'une réaction d'hybridation entre la séquence à détecter et la sonde.
- avantageusement des réactifs permettant la détection des complexes d'hybridation formés entre la séquence de nucléotides et la sonde lors de la réaction d'hybridation.

Pour la mise en oeuvre des méthodes de dépistage in vitro définies ci-dessus, basées sur l'utilisation d'anticorps on aura recours avantageusement à des nécessaires ou kits, comprenant:
- une quantité déterminée d'un anticorps selon l'invention,
- avantageusement un milieu approprié à la formation d'une réaction immunologique entre au moins une partie de la protéine à activité uréase produite par une souche de C.pylori et l'anticorps,
- avantageusement, des réactifs permettant la détection des complexes immunologiques formés entre au moins une partie de la protéine à activité uréase et l'anticorps lors de la réaction immunologique.

Une étude approfondie du fragment de 4,2kb défini ci-dessus a permis de situer les gènes de structure codant pour les sous-unités polypeptidiques majeures de l'uréase de C.pylori.

Cette étude a été réalisée en construisant des mutants dans les plasmides recombinants introduits chez E.coli et C.jejuni incapables de synthétiser l'uréase, (Baskerville-A, Newell D. (1988), Gut.29, 465-472). Le système de transcription-traduction in vitro d'E.coli ainsi qu'un système d'expression en minicellules ont été utilisés afin d'identifier et de localiser, au sein du fragment de 4,2kb de C.pylori, l'ensemble des gènes codant pour les sous-unités de l'uréase: deux protéines majeures de 26kDa et 61kDa ont été identifiées. Parallèlement, après diverses délétions au sein du fragment de 4,2kb, un système de transfert par conjugaison d'une souche d'E.coli à C.jejuni (du type de celui explicité dans la description détaillée qui suit) a été utilisé pour identifier les régions indispensables à l'expression de l'activité uréasique et corréler la délétion d'une région avec la disparition d'un polypeptide et la perte de l'activité uréasique. Il a été ainsi déterminé que les gènes codant pour les deux polypeptides nécessaires à l'expression de l'activité uréasique se situent dans la région centrale de 03,35kb, en aval du site H3 de la figure 2, et à cheval sur B1. La transcription des gènes codant pour les polypeptides de 61kDa ou de 26kDa se fait dans le sens EcoRI-PstI. Le fragment de 3,35kb est nécessaire mais non suffisant à l'expression de l'activité uréasique chez C.jejuni. Une région adjacente de 0,85kb, située en amont et apparemment non associée à l'expression de polypeptides chez E.coli, est indispensable à l'expression de l'activité uréasique chez C.jejuni.

L'analyse des mutants de délétion par transcription-traduction in vitro a permis d'observer que les deux polypeptides de 26 et 61kDa sont codés par deux fragments d'ADN adjacents.

La séquence nucléotidique (V) suivante est également décrite ci-après:

1
ATGAAACTCACCCCAAAAGAGTTAGATAAGTTGATGCTCCACTACGCTGGAGAATTGGCT
AAAAAACGCAAAGAAAAAGGCATTAAGCTTAACTATGTAGAAGCAGTAGCTTTGATTAGT
GCCCATATTATGGAAGAAGCGAGAGCTGGTAAAAAGACTGCGGCTGAATTGATGCAAGAA
GGGCGCACTCTTTTAAAACCAGATGATGTGATGGATGGCGTGGCAAGCATGATCCATGAA
GTGGGTATTGAAGCGATGTTTCCTGATGGGACTAAACTCGTAACCGTGCATACCCCTATT
GAGGCCAATGGTAAATTAGTTCCTGGTGAGTTGTTCTTAAAAAATGAAGACATCACTATC
AACGAAGGCAAAAAAGCCGTTAGCGTGAAAGTTAAAAATGTTGGCGACAGACCGGTTCAA
ATCGGCTCACACTTCCATTTCTTTGAAGTGAATAGATGCCTAGACTTTGACAGAGAAAAA
ACTTTCGGTAAACGCTTAGACATTGCGAGCGGGACAGCGGTAAGATTTGAGCCTGGCGAA
GAAAAATCCGTAGAATTGATTGACATTGGCGGTAACAGAAGAATCTTTGGATTTAACGCA
TTGGTTGATAGACAAGCAGACAACGAAAGCAAAAAAATTGCTTTACACAGAGCTAAAGAG
CGTGGTTTTCATGGCGCTAAAAGCGATGACAACTATGTAAAAACAATTAAGGAGTAA
717

Plus particulièrement, la présente demande décrit une séquence nucléotidique correspondant, selon le code génétique universel à la séquence en acides animés (VI) de 26kDa (codée par la séquence nucléotidique (V)) suivante: 
1

MKLTPKELDKLMLHYAGELAKKRKEKGIKLNYVEAVALISAHIMEEARAGKKTAAELMQE

GRTLLKPDDVMDGVASMIHEVGIEAMFPDGIKLVTVHIPIEANGKLVPGELFLKNEDITI

NEGKKAVSVKVKNVGDRPVQIGSHFHFFEVNRCLDFDREKTFGKRLDIASGTAVRFEPGE

EKSVELIDIGGNRRIFGFNALVDRQADNESKKIALHRAKERGFHGAKSDDNYVKTIKE
239
 239 acides aminés
Poids moléculaire : 26.522 daltons

La séquence nucléotidique (VII) suivante code pour la séquence en acides aminés (VIII).

1
ATGAAAAAGATTAGCAGAAAAGAATATGTTTCTATGTATGGTCCTACTACAGGCGATAAA
GTGAGATTGGGCGATACAGACTTGATCGCTGAAGTAGAACATGACTACACCATTTATGGC
GAAGAGCTTAAATTCGGTGGCGGTAAAACCCTAAGAGAAGGCATGAGCCAATCTAACAAC
CCTAGCAAAGAAGAGTTGGATTTAATTATCACTAACGCTTTAATCGTGGATTACACCGGT
ATTTATAAAGCGGATATTGGTATTAAAGATGGCAAAATCGCTGGCATTGGTAAAGGCGGT
AACAAAGACATGCAAGATGGCGTTAAAAACAATCTTAGCGTAGGTCCTGCTACTGAAGCC
TTAGCCGGTGAAGGTTTGATCGTAACGGCTGGTGGTATTGACACACACATCCACTTCATT
TCACCCCAACAAATCCCTACAGCTTTTGCAAGCGGTGTAACAACCATGATTGGTGGTGGA
ACCGGTCCTGCTGATGGCACTAATGCGACTACTATCACTCCAGGCAGAAGAAATTTAAAA
TGGATGCTCAGAGCGGCTGAAGAATATTCTATGAATTTAGGTTTCTTGGCTAAAGGTAAC
GCTTCTAACGATGCGAGCTTAGCCGATCAAATTGAAGCCGGTGCGATTGGCTTTAAAATT
CACGAAGACTGGGGCACCACTCCTTCTGCAATCAATCATGCGTTAGATGTTGCGGACAAA
TACGATGTGCAAGTCGCTATCCACACAGACACTTTGAATGAAGCCGGTTGTGTAGAAGAC
ACTATGGCTGCTATTGCTGGACGCACTATGCACACTTTCCACACTGAAGGCGCTGGC[?]GC
GGACACGCTCCTGATATTATTAAAGTAGCCGGTGAACACAACATTCTTCCCGCTTCCACT
AACCCCACCATCCCTTTCACCGTGAATACAGAAGCAGAGCACATGGACATGCTTATGGTG
TGCCACCACTTGGATAAAAGCATTAAAGAAGATGTTCAGTTCGCTGATTCAAGGATCCGC
CCTCAAACCATTGCGGCTGAAGACACTTTGCATGACATGGGGATTTTCTCAATCACCAGT
TCTGACTCTCAAGCGATGGGCCGTGTGGGTGAAGTTATCACTAGAACTTGGCAAACAGCT
GACAAAAACAAGAAAGAATTTGGCCGCTTGAAAGAAGAAAAAGGCGATAACGACAACTTC
AGGATCAAACGCTACTTGTCTAAATACACCATTAACCCAGCGATCGCTCATGGGATTAGC
GAGTATGTAGGTTCAGTAGAAGTGGGCAAAGTGGCTGACTTGGTATTGTGGAGTCCAGCA
TTCTTTGGCGTGAAACCCAACATGATCATCAAAGGCGGATTCATTGCGTTAAGCCAAATG
GGCGATGCGAACGCTTCTATCCCTACCCCACAACCGGTTTATTACAGAGAAATGTTCGCT
CATCATGGTAAAGCTAAATACGATGCAAACATCACTTTTGTGTCTCAAGCGGCTTATGAC
AAAGGCATTAAAGAAGAATTAGGACTTGAAAGACAAGTGTTGCCGGTAAAAAATTGCAGA
AATATCACTAAAAAAGACATGCAATTCAACGACACTACTGCTCACATTGAAGTCAATCCT
GAAACTTACCATGTGTTCGTGGATGGCAAAGAAGTAACTTCTAAACCAGCCAATAAAGTG
AGCTTGGCGCAACTCTTTAGCATTTTCTAG
1710

L'invention a plus particulièrement pour objet toute séquence nucléotidique correspondant, selon le code génétique universel à au moins une partie de la séquence en acides aminés (VIII) de 61kDa (codée par la séquence nucléotidique (VII)) suivante: 
1
MKKISRKEYVSMYGPTTGDKVRLGDTDLIAEVEHDYTIYGEELKFGGGKTLREGM
SQSNNPSKEELDLIITNALIVDYTGIYKADIGIKDGKIAGIGKGGNKDMQDGVKNN
LSVGPATEALAGEGLIVTAGGIDTHIHFISPQQIPTAFASGVTTMIGGGTGPAD
GTNATTITPGRRNLKWMLRAAEEYSMNLGFLAKGNASNDASLADQIEAGAIGF
KIHEDWGTTPSAINHALDVADKYDVQVAIHTDTLNEAGCVEDTMAAIAGRTMH
TFHTEGAGGGHAPDIIKVAGEHNILPASTNPTIPFTVNTEAEHMDMLMVCHHLD
KSIKEDVQFADSRIRPQTIAAEDTLHDMGIFSITSSDSQAMGRVGEVITRTWQT
ADKNKKEFGRLKEEKGDNDNFRIKRYLSKYTINPAIAHGISEYVGSVEVGKVADL
VLWSPAFFGVKPNMIIKGGFIALSQMGDANASIPTPQPVYYREMFAHHGKAKYD
ANITFVSQAAYDKGIKEELGLERQVLPVKNCRNITKKDMQFNDTTAHIEVNPETY
HVFVDGKEVTSKPANKVSLAQLFSIF
569
 569 Acides aminés
Poids moléculaire : 61 729 daltons

La comparaison de la séquence en acides aminés VIII avec celle de l'uréase d'haricot (Jack Bean) décrit notamment dans Eur. J. Biochem 175, 151-165 (1588), montre de façon inattendue un haut niveau d'homologies d'acides aminés. Cette conservation de structure a permis aux inventeurs de déduire le site actif de la sous-unité uréasique de 61kDa qui est compris, en totalité ou en partie, dans la séquence polypeptidique délimitée par les acides aminés situés aux positions 206 et 338 de la séquence VIII décrite ci-dessus.

La présente demande décrit également toute ou partie de la séquence nucléotidique IX constituée successivement de la

La demande decrit également une séquence nucléotidique correspondant, selon le code génétique universel à au moins une partie de la séquence en acides aminés (X) de 87kDa constituée successivement de la séquence VI et de la séquence VIII.

La demande décrit également des sondes de détection de la présence éventuelle de C.pylori dans un échantillon biologique, caractérisée en ce qu'elles comprennent au moins une partie d'une séquence de nucléotides définies ci-dessus.

Il va de soi que les bases des séquences de nucléotides considérées peuvent être dans un ordre différent de celui trouvé dans les gènes et/ou que ces bases peuvent être, le cas échéant, substituées, dès lors qu'une sonde élaborée à partir de telles séquences donne une réponse caractéristique et non équivoque quant à la capacité de reconnaître la présence de gènes codant pour la protéine à activité uréase de C.pylori.

Toute séquence de nucléotides hybridable avec celle des enchaînements de nucléotides sus-mentionnés telle qu'obtenue par transcription enzymatique inverse de l'ARN correspondant ou encore par synthèse chimique, entre également dans le cadre de l'invention.

Toute sonde ne se distiguant de la précédente, au niveau de sa séquence de nucléotides, que par des substitutions ou altérations de nucléotides n'entraînant pas de modification de ses propriétés d'hybridation avec le génome de C.pylori, entre dans le cadre de l'invention.

Les sondes sus-mentionnées sont utilisables dans des procédés de diagnostic in vitro de la présence éventuelle de C.pylori dans un échantillon biologique décrits ci-dessus.

L'invention a également pour objet des amorces nucléotidiques d'environ 10 à environ 40 nucléotides, ces amorces étant comprises dans l'une des séquences nucléotidiques V ou VII définies ci-dessus (ou dans l'une des séquences dérivées de ces séquences V et VII), ou étant susceptibles de s'hybrider avec une partie d'une des séquences nucléotidiques sus-mentionnées ou avec leurs séquences complémentaires (notamment dans les conditions d'hybridation définies ci-dessus).

L'invention concerne également l'utilisation de ces amorces dans les méthodes de diagnostic in vitro définies ci-dessus, pour l'amplification préalable de la quantité de nucléotides de C.pylori susceptibles d'être contenus dans l'échantillon biologique selon le procédé décrit ci-dessus.

L'invention se rapporte également aux vecteurs recombinants d'expression et de clonage, capables de transformer une cellule hôte appropriée comportant toute ou partie d'une séquence nucléotidique identique ou dérivée des séquences nucléotidiques V, VII et IX sus-mentionnées.

L'invention vise également un procédé de production de protéines correspondant à tout ou partie de la séquence X sus-mentionnée, ce procédé comprenant la transformation de cellules hôtes appropriées (notamment celles décrites ci-dessus) à l'aide des vecteurs sus-mentionnés et la récupération des protéines ainsi produites suivie, le cas échant, par une étape de purification de ces protéines.

Le procédé de production de protéines défini ci-dessus comprend le cas échéant une étape préalable d'amplification de gène codant pour la protéine dont la production est recherchée, cette étape étant réalisée à l'aide de couples d'amorces de l'invention selon le procédé décrit ci-dessus.

L'invention a également pour objet les polypeptides (ou protéines) codés par les séquences nucléotidiques décrites ci-dessus. En particulier l'invention vise la séquence en acides aminés X de 87kDa, et ses fragments, lesdits fragments n'étant pas un fragment peptidique de la séquence (VI ou VIII).

L'invention concerne également les anticorps polyclonaux et monoclonaux obtenus à partir de toutes ou partie des polypeptides décrits ci-dessus et susceptibles de former un complexe immunologique avec eux.

L'invention vise particulièrement les anticorps, notamment monoclonaux, obtenus à l'aide d'une séquence peptidique d'au moins environ 10 à 15 acides aminés de la séquence VI, ou de la séquence VIII.

Des anticorps monoclonaux préférés de l'invention sont dirigés contre toute ou partie de la séquence délimitée par les acides aminés situés aux positions 206 et 338 de la séquence VIII, et plus particulièrement ceux obtenus à l'aide d'une séquence peptidique d'au moins environ 10 à 15 acides aminés issue de la séquence délimitée par les acides aminés situés aux positions 206 et 338 de la séquence VIII.

L'invention a également pour objet l'utilisation des anticorps sus-mentionnés pour la mise en oeuvre de méthodes de diagnostic in vitro ainsi que dans des kits de diagnostic in vitro de l'infection d'un individu par C.pylori tels que décrits ci-dessus.

L'invention vise également des compositions immunogènes comprenant toute ou partie des polypeptides décrits ci-dessus, et plus particulièrement, toute ou partie de la séquence polypeptidique délimitée par les acides aminés situés aux positions 206 et 338 de la séquence VIII, en association avec un véhicule pharmaceutiquement acceptable.

De telles compositions immunogènes sont utilisables en tant que compositions de vaccin pour la prévention de l'infection d'un individu par C.pylori.

L'invention concerne également des compositions pharmaceutiques comprenant un (ou plusieurs) anticorps tels que décrits ci-dessus, et plus particulièrement des anticorps susceptibles de former un complexe immunologique avec toute ou partie de la séquence peptidique délimitée par les aminés situés aux positions 206 et 338 de la séquence VIII, en association avec un véhicule pharmaceutiquement acceptable.

De telles compositions pharmaceutiques selon l'invention sont utilisables par le traitement des pathologies liées à l'infection d'un individu par C.pylori, notamment des gastrites, et des ulcères gastriques et duodénaux.

D'autres avantages et caractéristiques de l'invention sont rapportés dans la description qui suit concernant le clonage des gènes responsables de la production de la protéine à activité uréase chez C.pylori, le séquençage de la région associée à l'expression de la protéine à activité uréase et la spécificité de sondes nucléotidiques pour la détection de C.pylori.

Dans cette description, on se réfère aux figures 1 et 2 qui représentent:
- la figure 1, la carte de restriction d'un fragment PstI-EcoRI du cosmide recombinant IID2 portant l'information requise pour l'expression de l'uréase chez C.jejuni, et
- la figure 2, la carte de restriction d'un fragment d'environ 8kb de pILL590.

### 1) Réalisation d'une banque génomique dans un vecteur cosmidique navette; clonage des gènes impliqués dans la production de l'uréase chez C.pylori.

L'ADN chromosomique de la souche C.pylori (85P) est préparé de façon à générer des fragments d'ADN de haut poids moléculaire; environ 300µg de cet ADN chromosomique sont soumis à une digestion partielle contrôlée par l'endonucléase de restriction Sau3A et déposés sur gradient de sucrose de façon à isoler sélectivement des fragments de digestion compris entre 35 et 45 kilobases. 1,5µg de ces fragments sont ligaturés in vitro au vecteur navette-cosmide pILL575 linéarisé par l'endonucléase BamHI et déphosphorylé. pILL575 a été construit à partir du cosmide navette pILL550 décrit par Labigne-Roussel et al. dans J. Bacteriol. 169:5320-5323, 1987, dans lequel a été inséré le site "cos" du phage lambda (fragment Bgl II de 1,7kb de pERG153 inséré au site Pvu II de pILL550); il est donc comme lui mobilisable par conjugaison, code pour une résistance à la kanamycine qui s'exprime à la fois chez E.coli et Campylobacter (Km^{r}) et se réplique à la fois chez E.coli et chez C.jejuni du fait de la présence de deux origines de réplication, l'une fonctionnelle chez E.coli exclusivement l'autre uniquement chez Campylobacter. Les produits de cette ligation sont empaquetés in vitro dans les particules de lambda puis introduits par transfection dans une souche de E.coli hébergeant un plasmide IncP capable de complémenter en trans les fonctions de transfert des cosmides recombinants. Les colonies de E.coli infectées et résistantes à la kanamycine sont immédiatement conservées individuellement à -80°C; une banque génomique cosmidique représentative de l'ensemble du génome de Campylobacter pylori est réalisée chez E.coli en conservant environ 500 clones indépendants.

Chacun des cosmides recombinants est alors transféré par conjugaison d'E.coli à une souche réceptrice de Campylobacter jejuni C31, naturellement urease.

On vérifie pour chacun de ces transconjugants résistants à la kanamycine, s'il y a synthèse par Campylobacter jejuni d'une uréase.

On isole un cosmide sur 106 analysés portant une information génétique conférant à Campylobacteur jejuni C31 la capacité de produire une uréase. Le cosmide recombinant (IID2) a une taille de 54 kilobases. La position relative des sites de clivage par les endonucléases de restriction PstI, BamHI, SmaI et EcoRI sur cette séquence est présentée sur la figure 1.

### 2) Identification du ou des gènes requis pour la production d'uréase chez Campylobacter pylori.

On effectue le sous-clonage des gènes uréases en réalisant à partir de l'ADN cosmidique IID2 préparé chez E.coli des digestions partielles par l'endonucléase Sau3A de façon à générer des fragments de digestions partielles allant de 8 à 15 kilobases. On recherche le plus petit plasmide hybride cloné chez E.coli conférant l'aptitude à produire une uréase après son transfert par conjugaison à Campylobacter récepteur.

4 plasmides sur 37 testés portent l'information nécessaire à l'expression de l'uréase chez Campylobacter jejuni: pILL586, pILL589 et pILL590 (cf. figure 2).

Par comparaison des cartes de restriction de ces quatre hybrides, on constate la présence d'une séquence de nucléotides de 4,2 kilobases commune aux quatre hybrides, suggérant qu'il s'agit là de la région requise pour l'expression de l'uréase. Un certain nombre de délétions ont été réalisées qui confirme ces conclusions (cf. figure 2).

Le fragment EcoRI-Pst I de pILL590 a été utilisé comme sonde dans des hybridations avec l'ADN total de C.pylori 85P en vue de s'assurer que les différents fragments de restrictions générés avec BamHI et HindIII à partir de pILL590 étaient présents dans 85P. Les résultats obtenus montrent que la séquence de 8kb présente dans pILL590 n'a pas subi de réarrangements au cours des différentes étapes de clonage.

### 3) Séquence de nucléotides de la région associée à l'expression de l'uréase.

La séquence du fragment HindIII compris entre les sites H2 et H3 correspond à la séquence des 294 nucléotides de l'enchaînement (I).

La séquence suivante de 610 nucléotides de l'enchaînement (II) est interne au fragment défini par les sites H4 et B1 (figure 2).

### 4) Spécificité des sondes nucléotidiques pour la détection de Campylobacter pylori.

Le fragment EcoRI-PstI de 8kb provenant de pILL590 a été utilisé comme sonde radioactive dans des expériences d'hybridation.

32 Campylobacter pylori ont été testés en hybridation sur colonies et ont donné un signal positif; sur 35 Campylobacter jejuni, fétus et coli étudiés aucune n'a donné de signal positif dans des conditions d'hybridation stringentes classiques (50% de formamide, 37°, 3XSSC).

Dans ces mêmes conditions, la sonde C.pylori de 8 kilobases n'a pas donné de signal positif avec la liste des microorganismes suivants qui sont tous naturellement des producteurs d'uréase:

Klebsiella oxytoca (3 souches), Klebsiella pneumoniae (3 souches), Proteus mirabilis (3 souches) Proteus morganii (3 souches) Proteus vulgaris (2 souches) Providencia stuartii (3 souches), Pseudomonas picketti (3 souches), Yersinia enterocolitica (3 souches), 5 souches d'Acinetobacter uréase⁺, 2 souches d'Escherichia coli uréase⁺ et 3 souches de Citrobacter freundii uréase⁺.

L'absence de faux positif avec le fragment 8kb garantit la spécificité des sondes nucléotidiques proposées.

Un procédé de préparation d'anticorps comprend:
- l'immunisation d'un animal à l'aide d'un polypeptide ci-dessus défini, et
- la récupération des anticorps formés selon les techniques classiques.

Un mode de préparation approprié des acides nucléiques (comportant au maximum 200 nucléotides - ou pb, lorsqu'il s'agit d'acides nucléiques bicaténaires) de l'invention par voie chimique comprend les étapes suivantes:
- la synthèse d'ADN en utilisant la méthode automatisée des bêta-cyanethyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides - ou pb (lorsqu'il s'agit d'acides nucléiques bicaténaires) comprend les étapes suivantes:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

## Revendications

1. Séquence de nucléotides correspondant, selon le code génétique universel, à la séquence comprise entre les acides aminés situés aux positions 206 et 338 de la séquence (VIII).

2. Séquence de nucléotides **caractérisée en ce qu'**elle est délimitée par deux sites de restrictions de la figure 2 compris entre les sites H1 et B2 et comprenant les sites H1, H2, H3, H4, B1 et B2.

3. Fragments peptidiques **caractérisés en ce qu'**ils sont choisis parmi :
a) La séquence an acides aminés (X) de 87 KDa
b) Un fragment de la séquence a), ledit fragment comportant une activité uréase et n'étant pas un fragment peptidique de la séquence (VI) ou (VIII).

4. Procédé de production d'un complexe immunologique entre une protéine à activité uréase exprimée par *C. pylori* ou des fragments de cette demière conformément à ceux de la revendication 3 et des anticorps, ledit procédé étant **caractérisé par** la mise en contact de ladite protéine ou desdits fragments peptidiques avec un liquide biologique contenant lesdits anticorps.

5. Utilisation des séquences selon les revendications 1 et 2 comme sonde de détection dans un procédé de dépistage in vitro de la présence de *C. pylori* dans un échantillon susceptible de le contenir.

6. Utilisation des fragments selon la revendication 3 dans la fabrication d'une composition pour l'induction de la synthèse d'anticorps spécifiques contre lesdits fragments ou d'une réponse immunitaire.

7. Utilisation des fragments peptidiques choisis parmi a) la séquence en acides aminés (VI) de 26 kDa, b) la séquence en acides aminés (VIII) de 61 kDa ou c) un fragment de l'une des séquences a) ou b), ledit fragment comportant une activité uréase, dans la fabrication d'une composition pour l'induction de la synthèse d'anticorps spécifiques contre lesdits fragments ou d'une réponse immunitaire.

## Patentansprüche

1. Nukleotidsequenz, die gemäß dem universellen genetischen Code der Sequenz entspricht, die zwischen den Aminosäuren an den Positionen 206 und 338 der Sequenz (VIII) liegt.

2. Nukleotidsequenz, **dadurch gekennzeichnet, dass** sie durch 2 Restriktionsstellen aus Figur 2, die zwischen den Stellen H1 und B2 liegen und die Stellen H1, H2, H3, H4, B1 und B2 umfassen, begrenzt wird.

3. Peptidfragmente, **dadurch gekennzeichnet, dass** sie ausgewählt werden aus
a) der Aminosäuresequenz (X) mit 87 kDa
b) einem Fragment der Sequenz a), wobei das Fragment eine Urease-Aktivität aufweist und kein Peptidfragment der Sequenz (VI) oder (VIII) ist.

4. Verfahren zur Herstellung eines immunologischen Komplexes zwischen einem Protein mit Urease-Aktivität, exprimiert durch *C. pylori*, oder Fragmenten davon gemäß jenen nach Anspruch 3 und Antikörpern, wobei das Verfahren **dadurch gekennzeichnet ist, dass** das Protein oder die Peptidfragmente mit einer biologischen Flüssigkeit, die die Antikörper enthält, in Kontakt gebracht wird bzw. werden.

5. Verwendung der Sequenzen gemäß den Ansprüchen 1 und 2 als Detektionssonde in einem Verfahren zum in vitro Nachweis im Hinblick auf das Vorliegen von *C. pylori* in einer Probe, die dieses enthalten könnte.

6. Verwendung der Fragmente gemäß Anspruch 3 bei der Herstellung einer Zusammensetzung zur Induktion der Synthese von spezifischen Antikörpern gegen die Fragmente oder einer Immunantwort.

7. Verwendung der Peptidfragmente, ausgewählt aus a) der Aminosäuresequenz (VI) mit 26 kDa, b) der Aminosäuresequenz (VIII) mit 61 kDa oder c) einem Fragment der Sequenzen a) oder b), wobei das Fragment eine Urease-Aktivität aufweist, bei der Herstellung einer Zusammensetzung zur Induktion der Synthese von spezifischen Antikörpern gegen die Fragmente oder einer Immunantwort.

## Claims

1. A nucleotide sequence corresponding, in accordance with the universal genetic code, to the sequence included between the amino acids located at positions 206 and 338 of sequence (VIII).

2. A nucleotide sequence, **characterized in that** it is delimited by two restriction sites of Figure 2 included between sites H1 and B2 and including sites H1, H2, H3, H4, B1 and B2.

3. Peptide fragments, **characterized in that** they are selected from:
a) the 87 kDa amino acid sequence (X);
b) a fragment of sequence a), said fragment having a urease activity and not being a peptide fragment of sequence (VI) or (VIII).

4. A method for producing an immunological complex between a protein with urease activity expressed by *C. pylori* or fragments of the latter in accordance with claim 3 and antibodies, said method being **characterized by** bringing said protein or said peptide fragments into contact with a biological liquid containing said-antibodies.

5. Use of sequences according to claims 1 and 2 as a probe for detecting the presence of *C. pylori*, in an in vitro detection method, in a sample that is suspected of containing it.

6. Use of fragments according to claim 3 in the manufacture of a composition for inducing the synthesis of specific antibodies against said fragments or an immune response.

7. Use of peptide fragments selected from a) the 26 kDa amino acid sequence (VI); b) the 61 kDa amino acid sequence (VIII); and c) a fragment of one of sequences a) or b), said fragment having a urease activity, in the manufacture of a composition for inducing the synthesis of specific antibodies against said fragments or an immune response.
